# NEUE EUROPÄISCHE PATENTSCHRIFT

(11) **EP 0 291 632 B2**
(45) Veröffentlichungstag und Bekanntmachung des Hinweises auf die Entscheidung über den Einspruch: **12.08.1998**
(45) Hinweis auf die Patenterteilung: 21.07.1993
(21) Anmeldenummer: 88101736.2
(22) Anmeldetag: 06.02.1988
(51) Int. Cl.: A61B 17/58

(54) **Kleinknochenplatte, insbesondere für die Versorgung von Frakturen des Schädel- und Gesichtsskeletts oder dergleichen**
Small bone plate, in particular for the treatment of skull and facial bone fractures
Plaquette d'ostéosynthèse, en particulier pour le traitement de fractures des os du crâne et de la face

(30) Priorität: 14.05.1987 DE 8706912 U
(43) Veröffentlichungstag der Anmeldung: 23.11.1988
(73) Patentinhaber: Howmedica GmbH, D-24232 Schönkirchen (DE)
(72) Erfinder: Luhr, Hans-Georg, Prof. Dr. Dr., D-3400 Göttingen (DE); Harder, Hans Erich, D-2316 Probsteierhagen (DE)
(74) Vertreter: Patentanwälte Hauck, Graalfs, Wehnert, Döring, Siemons

(56) Entgegenhaltungen:
- EP-A- 236 698
- DE-A- 3 329 287
- DE-U- 8 528 003
- GB-A- 2 125 295
- US-A- 370 255
- US-A- 2 494 229
- US-A- 3 913 647
- US-A- 5 372 598
- BIOMEDIZINISCHE TECHNIK, Band 31, Nr. 12, Dezember 1986, Seiten 303-307, Berlin, DE; TH. HEINL et al.: "Vorschlag für die analytisch begründete Konstruktion einer Osteosynthese-Miniplatte aus Titan"
- Sherman: "Vanadium Steel Bone Plates and Screws"; Surgery, Gynecology and Obstetrics, Volume XIV, 1912, Seiten 629 bis 634
- TH. Heinl et al: "Vorschlag für die analytisch begründete Konstruktion einer Osteosynthese-Miniplatte as Titan"; Biomedizinische Technik, Band 31, Nr. 12, Dez. 1986, Seiten 303 bis 307
- Katalog "Instrumente zur Mund-, Kiefer- und Gesichtschirurgie" der Firma Medicon e.G.; 4. Quartal 1974
- Katalog Nr. 12 "Chirurgie" der Firma Medicon e.G.; 1985
- Katalog "Implantate, Instrumente und Motoren zur Frakturbehandlung und Orthopädie" der Firma Aesculap-Werke AG, Juni 1978
- "A Discussion of Relevant Issues Concerning Materials used in Maxillofacial Plating Systems"
- Katalog "Würzburg Titan-Miniplatten-Systems" der Firma Oswald Leibinger GmbH; 12/85

## Beschreibung

Die Erfindung bezieht sich auf eine Kleinknochenplatte, insbesondere für die Versorgung von Frakturen des Schädel- und Gesichtsskeletts gemäß dem Oberbegriff des Patentanspruchs 1.

Derartige Knochenplatten sind etwa aus dem DE-U-85 28 003 bekanntgeworden. Sie bestehen aus einem körperverträglichen Material, beispielsweise Titan oder einer Chromkobaltmolybdänlegierung. Die relativ dünnen Platten sind streifenförmig gestaltet und in Abständen mit Löchern zur Aufnahme von Knochenschrauben versehen. Sie sind so ausgelegt, daß sie vom Chirurgen verformt werden können, um sie dem Knochen im zu versorgenden Bereich anzupassen. Es ist auch bekanntgeworden, zwischen den Schraublöchern dünne Stege vorzusehen und sie in der Querschnittsfläche so auszulegen, daß die Stegquerschnittsfläche kleiner ist als das Zweifache der Querschnittsfläche der Lochumrandung. Auf diese Weise soll sichergestellt werden, daß bei einer Anpassung der Knochenplatte an die Konturen des Knochen die Lochumrandung nicht verbogen wird. Vielmehr soll der Biegewiderstand im Bereich der Stege kleiner sein als im Bereich der Schraublochumrandung, so daß im wesentlichen nur eine Verbiegung der Stege stattfindet. Es hat sich indessen gezeigt, daß die Stege einerseits eine gewisse Mindestfestigkeit aufweisen müssen, andererseits die Breite der Platten im Bereich der Schraublochumrandung bestimmte Werte nicht überschreiten darf. Es besteht daher gleichwohl die Gefahr, daß bei einer Verbiegung der Knochenplatten, insbesondere bei sehr kleinen Dimensionen, die Schraublochumrandung mitverformt wird.

Der Erfindung liegt daher die Aufgabe zugrunde, eine Kleinknochenplatte, insbesondere für die Versorgung von Frakturen des Schädel- und Gesichtsskeletts oder dergleichen zu schaffen, die eine leichte Verbiegung der Platten in ihrer Ebene zuläßt, ohne eine merkliche Verformung der Schraublochumrandungen zu verursachen.

Diese Aufgabe wird durch die Merkmale des Patentanspruchs 1 gelöst.

Bei der Erfindung ist erkannt worden, daß ein sehr kleiner Radius für den Überang vom Steg zur Schraublochumrandung zu wählen ist. Theoretisch wäre ein Übergang am günstigsten, bei dem die Seiten des Steges annähernd im rechten Winkel zur Schraublochumrandung verlaufen. Bei einem derartigen Übergang findet im wesentlichen keine Kraftübertragung vom Steg auf die Lochumrandung statt, wenn die Platte in ihrer Ebene gebogen wird. Derartige rechte Winkel sind schwierig herzustellen und wegen ihrer Kerbwirkung auch nicht erwünscht. In jedem Fall wird ein sehr kleiner Radius angestrebt, der zum Beispiel bei einer Stegbreite von 1,0 bis 1,5 mm und einer Dicke der Platte von 0,7 mm etwa 0,5 oder kleiner ist. Bei einer noch kleineren Platte, die eine Stegbreite von 0,5 bis 0,7 mm aufweist, ist dieser Radius 0,3 mm oder kleiner.

Bei der Verbiegbarkeit einer derartigen Platte spielen auch ihre Dicke und der Abstand der Schraublöcher naturgemäß eine Rolle.

Bei der erfindungsgemäßen Knochenplatte wird durch ihre Auslegung verhindert, daß bei einer Biegung der Knochenplatte in ihrer Ebene eine merkliche Beeinträchtigung der Schraublochumrandung stattfindet mit der Gefahr einer Sitzverschlechterung für den Schraubenkopf. Eine Verformung der Schraublochumrandung kann bei bekannten Knochenplatten so weit gehen, daß das Einsetzen der Schraube unmöglich gemacht wird.

Bekannte Knochenplatten bestehen aus mehrereren Schenkeln, die im Winkel zueinander angeordnet sind, beispielsweise L-förmige T- oder Doppel-T-förmige Knochenplatten. In diesem Zusammenhang sieht eine Ausgestaltung der Erfindung vor, daß ein Schenkel mit annähernd parallelen Seitenkanten versehen ist, der über einen verjüngten Steg in die Lochumrandung eines benachbarten Schenkels übergeht. Bei dieser Ausführungsform hat der eine Schenkel über einen großen Teil seiner Länge eine konstante Breite, welche dem Außendurchmesser der Schraublochumrandung entspricht. Ein derartiger Knochenplattenabschnitt ist daher verhältnismäßig stabil, während die übrigen Abschnitte im Sinne der Erfindung zwischen den Schraublochumrandungen verformbar sind. Der stabile Abschnitt dient mithin als statisches Element zur Verbindung von Fraktursegmenten, während die übrigen Knochenplattenabschnitte eine erleichterte Verformbarkeit durch den Chirurgen ermöglichen. Sie sind somit zwangsläufig auch mehr oder weniger dynamisch.

Etliche Partien des Gesichtsskeletts weisen eine äußerst geringe Weichteildeckung auf. Die beschriebenen Knochenplatten zur Versorgung von Frakturen des Gesichtsskeletts können langzeitig implantiert bleiben. Herkömmliche Platten sind so dimensioniert, daß sie durch Erhebungen im behandelten Bereich sichtbar sind. Um hier Abhilfe zu schaffen, hat die erfindungsgemäße Platte sehr kleine Abmessungen. Der Außendurchmesser der Lochumrandung beträgt vorzugsweise 2 bis 2,4 mm, insbesondere 2,2 mm. Damit ist eine Kleinstknochenplatte geschaffen, die so klein dimensioniert ist, daß sie im implantierten Zustand gar nicht oder kaum auftragend wirkt und auch bei dünner Weichteilbedeckung unauffällig bleibt.

Die Knochenplatten werden bekanntlich mit Hilfe von Schrauben in den Knochenfragmenten fixiert. Um einen ausreichend festen Sitz im Knochen zu erzielen, dürfen die Schraubenabstände nicht zu klein sein. Der relativ große Lochabstand ergibt einen langen Steg, der sich verhältnismäßig leicht verformen läßt, so daß die Platte während der Operation leicht der individuellen Knochenkontur angepaßt werden kann.

Die erfindungsgemäße Knochenplatte, insbesondere die Kleinstknochenplatte ist nicht auf die Anwendung im Bereich des Schädelgesichtsskeletts bei Erwachsenen und auch Kleinstkindern begrenzt. Beispielsweise können auch Kleinfragmente anderer Skelettabschnitte damit fixiert werden.

Die zur Fixierung der Knochenplatten verwendeten Schrauben sind üblicherweise mit einem Senkkopf versehen, der mit einer Ansenkung der Schraubenlöcher zusammenwirkt. Die Schrauben sind naturgemäß verhältnismäßig klein und daher entsprechend schwer zu handhaben. Eine Ausgestaltung der Erfindung sieht vor, daß die Schraube eine Linsen-Senkkopfschraube ist mit Kreuzschlitz und der Boden der Schlitze konkav gewölbt ist. Die Verwendung von Kreuzschlitzschrauben für Kleinknochenplatten ist an sich bekannt. Beim sogenannten Philips-Kreuzschlitz treten die Schlitze nicht über die Mantelfläche des Kopfes hinaus. Dadurch erhält man eine gewisse Zentrierwirkung. Nachteilig bei einem federartigen Kreuschlitz ist hingegen, daß die Schlitze sehr weit in den Schaft eintreten und diesen, insbesondere bei sehr geringen Durchmessern, unerwünscht stark schwächen. Außerdem muß ein Kopf mit einem Philips-Kreuzschlitz verhältnismäßig großvolumig sein, was ebenfalls bei sehr kleinen Knochenplatten nachteilig ist. Bei der anderen Kreuzschlitzart, dem sogenannten Sherman-Schlitz, treten die Schlitze über die Mantellinie aus, was zu einer entsprechenden Schwächung des Schraubenkopfes führt. Vor allen Dingen besteht die Gefahr, daß der Schraubendreher beim Verkanten der Schraube aus dem Kopf herausrutscht. Bei der erfindungsgemäßen Schraube ist der Grund der Schlitze konkav gewölbt. Dadurch wird zwar der Kopf etwas mehr als der Kopf beim Philips-Kreuzschlitz geschwächt, die Schwächung ist jedoch nicht in dem Maße gegeben wie beim Sherman-Schlitz, da die Schlitze bzw. die Wölbung ihres Grundes so gewählt werden können, daß sie oberhalb der Ansenkung aus dem Kopf austreten. Man erhält mithin nur einen gering geschwächten, jedoch relativ kleinvolumigen Kopf, dessen Außenfläche nur eine relativ schwache Wölbung aufweisen muß. Der Schraubenkopf trägt mithin nur sehr gering auf. Vor allen Dingen ist von großem Vorteil, daß durch die besondere Gestaltung des Kreuzschlitzes eine Selbstzentrierung beim Eingriff mit dem Schraubendreher stattfindet. Dieser ist nach einer weiteren Ausgestaltung der Erfindung vorzugsweise an den Enden konkav gewölbt, entsprechend der Wölbung des Schlitzgrundes.

Die bei den Kleinstplatten verwendete selbstschneidende Gewindeschraube ist nach einer weiteren Ausgestaltung der Erfindung mit einem Durchmesser von 0,5 bis 0,8 mm versehen, wobei der Kopf einen Durchmesser von etwa 1,6 mm aufweist. Die konkave Wölbung des Schlitzgrundes hat einen Radius von etwa 1,5 mm, während die konvexe Außenwölbung des Kopfes einen Radius von etwa 2 mm aufweist.

Die beschriebene Knochenschraube hat jedoch kein metrisches Gewinde, entsprechend dem Durchmesser von 0,8 mm, sondern sie ist mit einer Steigung von 0,25 bis 0,35 mm versehen, was im Fall von 0,25 der Steigung eines M1 Gewindes entspricht. Ferner ist erfindungsgemäß vorgesehen, daß der Kerndurchmesser nur etwa 0,4 mm beträgt. Auf diese Weise wird ein Gewinde erhalten, das einen festen und sicheren Sitz im Knochen gewährleistet. Insbesondere die relativ große Gewindetiefe führt zu einer wirksamen Verankerung im Knochen.

Die selbstschneidenden Eigenschaften werden bei den bekannten Schrauben durch eine oder zwei diametral gegenüberliegene Fräsnuten erhalten, die annähernd achsparallel im Bereich der Schraubenspitze eingearbeitet werden.

Darüber hinaus sieht eine Ausgestaltung der Erfindung vor, daß auf gegenüberliegenden Seiten des Gewindeschaftes nahe der Spitze Abflachungen vorgesehen sind, die in Richtung Spitze konvergieren. Im Bereich der Abflachungen, die durch Schleifen hergestellt werden, sind die Gewindegänge weggenommen. Im übrigen Bereich bleiben sie stehen. Eine derartige Ausformung stellt ausreichende Schneideigenschaften sicher, damit bei entsprechendem axialen Druck die Gewindeschraube ohne ein Vorbohren eines Loches leicht und wirksam mit dem Knochen verschraubt werden kann. Um ein erstes Eindringen der Schraube zu bewerkstelligen, sieht eine Ausgestaltung der Neuerung vor, daß eine Kegelspitze vorgesehen ist, vorzugsweise mit einem Winkel von etwa 60°.

Die Erfindung wird nachfolgend anhand von Zeichnungen näher erläutert.
- Fig. 1: zeigt schematisch eine Knochenschraube für Knochenplatten nach der Erfindung.
- Fig. 2: zeigt die Schraube nach Fig. 1 in Verbindung mit einer Knochenplatte.
- Fig. 3: zeigt eine Draufsicht auf eine erste Ausführungsform einer Knochenplatte nach der Erfindung.
- Fig. 4: zeigt eine Draufsicht auf eine zweite Ausführungsform einer Knochenplatte nach der Erfindung.
- Fig. 5: zeigt vergrößert die Schraube nach der Fig. 1.
- Fig. 6: zeigt eine dritte Ausführungsform einer Knochenplatte nach der Erfindung.

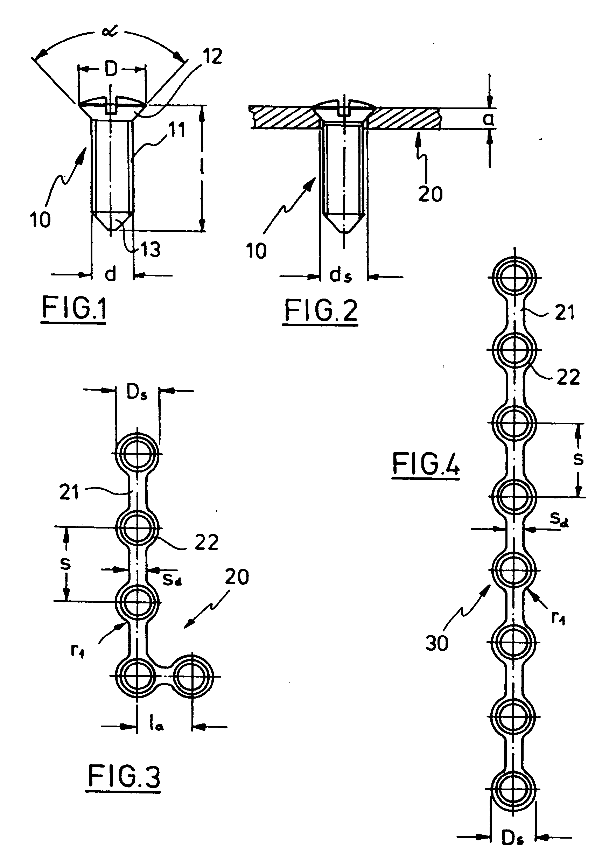

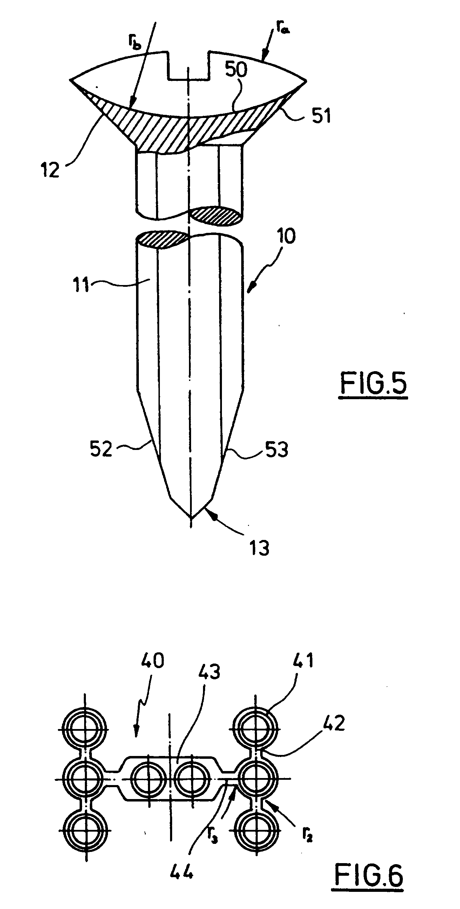

Die in Figuren 1 und 2 dargestellte Knochenschraube 10 besitzt einen Gewindeschaft 11, einen Kopf 12 sowie eine Spitze 13. Der Durchmesser d des Gewindeschafts 11 beträgt zum Beispiel 0,8 mm. Der Kopf 12 ist ein Linsen-Senkkopf mit einer Ansenkung im Winkel α = 90°. Der Durchmesser D des Kopfes beträgt etwa 1,6 mm. Auf weitere Einzelheiten der Schraube 10 wird in Verbindung mit Fig. 5 eingegangen.

In Fig. 3 ist eine L-förmige Knochenplatte 20 gezeigt. Sie setzt sich aus einzelnen Stegen 21 zusammen, zwischen denen jeweils eine Schraublochumrandung 22 angeordnet ist. Die Schraublochumrandungen haben einen Abstand S von etwa 3,5 mm. Die Stege haben eine Dicke von S_{D} von etwa 0,6 mm. Der Außendurchmesser der Schraublochumrandung D_{S} beträgt etwa 2,2 mm. Der Innendurchmesser d_{S} beträgt etwa 1,2 mm. Aus Fig. 2 geht der Querschnitt der Platte 20 hervor. Ihre Dicke a beträgt etwa 0,4 bis 0,5 mm. Man erkennt mithin, daß die Querschnittsfläche der Stege 21 kleiner ist als die zweifache Querschnittsfläche der Lochumrandung 22. Beim abgewinkelten Schenkel ergeben sich Lochabstände von 2,5 bzw. 4 mm. Es ist ferner wesentlich zu erwähnen, daß der Radius R1 zwischen dem Steg 21 und der Lochumrandung 22, d.h. der gerundete Übergang zwischen den Außenseiten der Stege 21 und der Lochumrandung 22 0,3mm oder weniger beträgt.

Die Dimensionen der Kleinstknochenplatte 30 gemäß Fig. 4 entsprechen denen nach Fig. 3, so daß sie im einzelnen nicht näher beschrieben werden sollen. Es handelt sich bei der Knochenplatte 30 um einen einzelnen längeren Streifen mit einer Reihe von Stegen 21 und Lochumrandungen 22.

Eine Knochenplatte 40 gemäß Fig. 6 weist etwas größere Abmessungen auf. Der Außendurchmesser der Lochumrandung 41 beträgt etwa 5 mm. Die dünneren Stege 42 zwischen den Schraublochumrandungen 41 haben eine Breite von 1 bis 1,5 mm. Die Knochenplatte 40 ist eine Doppel-T-Platte, wobei ihr Mittelsteg 43 über den größten Teil seiner Länge gleiche Breite aufweist, mithin nicht zwischen den Schraublochumrandungen verjüngt ist. Der Mittelsteg 43 geht in eingeschnürten schmaleren Stegen 44 von einer Breite von etwa 1,5 mm in die benachbarten Schraublochumrandungen 41 über. Die Dicke der Platte 40 beträgt etwa 0,7 mm. Der mittlere Abschnitt des Mittelstegs 43 ist daher verhältnismäßig stabil und wird bei der Verformung der übrigen Plattenabschnitte nicht mitverformt. Die Stege 42 bzw. 44 gehen ebenfalls in einem sehr kleinen Radius R2 bzw. R3 von etwa 0,5 mm in die Schraublochumrandung 41 über.

Bei beiden Ausführungsformen nach den Figuren 2 und 3 bzw. Fig. 6 bilden die Stege zwischen den Schraublochumrandungen annähernd einen Winkel mit der Schraublochumrandung im Anbindungsbereich.

Die in Fig. 5 vergrößert herausgestellte Schraube 10 ist mit einem Kreuzschlitz versehen, der einen konkav gekrümmten Boden 50 aufweist. Die Krümmung ist so gewählt, daß die Ansenkmantelfläche 51 nicht verletzt wird. Der Radius des Bodens r_{b} beträgt zum Beispiel 1,5 mm. Der Radius rₐ des Linsenkopfes 12 beträgt zum Beispiel 2 mm. Die Schlitzbreite ist 0,3 mm.

Die Spitze 13 ist kegelförmig angeschrägt in einem Winkel von 60°. An diese Spitze schließen sich auf gegenüberliegenden Seiten Abflachungen 52, 53 an, die in ihrem Bereich kein Gewinde aufweisen. Die Abflachungen 52, 53 und auch die Spitze 13 werden durch Schleifen geformt. Die Abflachungen 52, 53 stellen die selbstschneidende Eigenschaft der Schraube 10 sicher.

Die beschriebenen Teile bestehen aus einem körperverträglichen Material, insbesondere einer Chromkobaltmolybdänlegierung.

Es sei noch erwähnt, daß die Steigung des Gewindes der Schraube 10 0,25 beträgt, das Gewinde einen Flankenwinkel von 60° aufweist und der Kerndurchmesser etwa 0,4 mm beträgt.

## Patentansprüche

1. Kleinknochenplatte, insbesondere für die Versorgung von Frakturen des Schädel-Gesichtsskeletts oder dergleichen mit Schraublochumrandungen (22) zur Aufnahme von Knochenschrauben (10) und zwischen den Schraublochumrandungen (22) befindlichen verjüngten Stegen (21), wobei zumindest ein Teil der Stege (21) eine Querschnittsfläche aufweist, die gleich oder kleiner ist als das Zweifache der Querschnittsfläche der Schraublochumrandung (22) und wobei die Stege (21) in einem Kreisbogen in die Schraublochumrandungen (22) übergehen, dadurch gekennzeichnet, daß bei einem Mittenabstand (S) der Schraublochumrandungen (22) von 3,5 bis 5 mm und einer Plattendicke von 0,4 bis 0,7 mm bei einer Stegbreite von 0,5 bis 0,8 mm der Radius des Übergangs vom Steg (21) zur Schraublochumrandung (22) 0,3 mm oder kleiner und bei einer Stegbreite von 1,0 bis 1,5 mm und einer Dicke von 0,7 mm der Radius des Übergangs vom Steg (21) zur Schraublochumrandung (22) 0,5 mm oder kleiner ist.

2. Kleinknochenplatte nach Anspruch 1, bei der zwei oder mehrere Schenkel im Winkel zueinander angeordnet sind,dadurch gekennzeichnet, daß ein Schenkel (43) mit annähernd parallelen Seitenkanten versehen ist, der über einen verjüngten Steg (44) in eine Schraublochumrandung (41) eines benachbarten Schenkels übergeht.

3. Kleinknochenplatte nach Anspruch 1 oder 2, dadurch gekennzeichnet, daß die Kleinknochenplatte mit Schrauben versehen ist und die Schraube eine Linsen-Senkkopfschraube (10) ist mit Kreuzschlitz und der Boden (50) des Schlitzes konkav gewölbt ist.

4. Kleinknochenplatte mit Schrauben nach Anspruch 3, dadurch gekennzeichnet, daß der selbstschneidenden Gewindeschaft (11) der Schraube (10) einen Durchmesser von etwa 0,8 mm aufweist und der Kopf einen Durchmesser von 1,6 mm.

5. Kleinknochenplatte mit Schrauben nach Anspruch 3, dadurch gekennzeichnet, daß die konkave Wölbung einen Radius von etwa 1,5 und die konvexe Außenwölbung des Kopfes einen Radius von etwa 2 mm aufweist.

6. Kleinknochenplatte mit Schrauben nach einem der Ansprüche 3 bis 5, dadurch gekennzeichnet, daß das Gewinde bei einem Durchmesser von 0,8 mm eine Steigung von 0,25 bis 0,35 mm aufweist.

7. Kleinknochenplatte mit Schrauben nach einem der Ansprüche 3 bis 6, dadurch gekennzeichnet, daß der Kerndurchmesser der Schraube (10) etwa 0,4 mm beträgt.

8. Kleinknochenplatte mit Schrauben nach einem der Ansprüche 3 bis 7, bei der die Knochenschrauben ein selbstschneidendes Gewinde aufweisen, dadurch gekennzeichnet daß auf gegenüberliegenden Seiten des Gewindeschaftes (11) nahe der Spitze Abflachungen (52, 53) vorgesehen sind, die in Richtung Spitze (13) konvergieren.

9. Kleinknochenplatte mit Schrauben nach Anspruch 8, dadurch gekennzeichnet, daß eine Kegelspitze (13) vorgesehen ist, vorzugsweise mit einem Winkel von etwa 60°.

## Claims

1. A small bone plate, in particular for the treatment of skull and facial bone fractions or the like, comprising screw bore boundaries (22)for receiving bone screws (10) and reduced webs (21) between said screw bore boundaries (22), wherein at least a number of webs (21) has a cross-sectional area which is equal or smaller than two times the cross-sectional area of the screw bore boundary (22) and wherein said webs (21) arcuately join the screw bore boundaries (22), characterized in that for a center distance (S) of the screw bore boundaries (22) of 3.5 to 5.0 mm and a plat thickness of 0.4 to 0.7 mm for a web width of 0.5 to 0.8 mm the radius of the joining portions between the web (21) and the screw bore boundary (22) is 0.3 or less and for a web width of 1.0 to 1.5 mm and a thickness of 0.7 mm the radius of the joining portions between web (21) and screw bore boundary (22) is 0.5 mm or less.

2. The small bone plate according to claim 1, in which two or more legs are angled with respect to each other, characterized in that a leg (43) is provided with approximately parallel side edges joining a screw bone boundary (41) of an adjacent leg through a reduced web (44).

3. The small bone plate of one of claims 1 or 2, characterized in that the small bone plate is provided with screws and the screw is a cross-recessed raised counter-sunk head screw (10), wherein the bottom (50) of the recess is concavely curved.

4. The small bone plate including screws of claim 3, characterized in that the self-cutting thread portion (11) of the screw (10) has a diameter of about 0.8 mm and the head has a diameter of 1.6 mm.

5. The small bone plate including screws of claim 3, characterized in that the concave curvature has a radius of about 1.5 mm and the convex outer curvature of the head has a radius of about 2.0 mm.

6. The small bone plate including screws of one of claims 3 to 5, characterized in that the thread has a pitch of 0.25 to 0.35 mm for a diameter of 0.8 mm.

7. The small bone plate including screws of one of claims 3 to 6, characterized in that the core diameter of the screw (10) is about 0.4 mm.

8. The small bone plate including screws of one of claims 3 to 7, wherein the bone screws are provided with a self-cutting thread, characterized in that flats (52, 53) are provided close to the tip on opposite sides of the threaded portion (11) which flats converge towards the tip (13).

9. The small bone plate including screws of claim 8, characterized in that a conical tip (13) is provided, wherein the angle is preferably about 60°.

## Revendications

1. Plaquette pour la chirurgie des petits os, en particulier pour le traitement de fractures des os du crâne et de la face ou similaires, comportant des collerettes pour trous de vis (22) recevant des vis à os (10), et des entretoises amincies (21) qui se trouvent entre les collerettes de trous de vis (22), au moins une partie des entretoises (21) présentant une section dont l'aire est égale ou inférieure au double de l'aire de la section de la collerette pour trou de vis (22), et les barrettes (21) se raccordant en arc de cercle aux collerettes de trous de vis (22), caractérisée en ce que, pour une distance de centre à centre (S) des collerettes pour trous de vis (22) de 3,5 à 5 mm, une épaisseur de plaquette de 0,4 à 0,7 mm et une épaisseur d'entretoise de 0,5 à 0,8 mm, le rayon de la transition entre l'entretoise (21) et la collerette de trou de vis (22) est inférieur ou égal à 0,3 mm, et pour une largeur d'entretoise de 1,0 à 1,5 mm et une épaisseur de 0,7 mm, le rayon de la transition entre l'entretoise (21) et la collerette de trou à vis (22) est inférieure ou égale à 0,5 mm.

2. Plaquette selon la revendication 1, dans laquelle deux ou plusieurs branches sont disposées en angle les unes par rapport aux autres, caractérisée en ce qu'une branche (43) présente des bords latéraux approximativement parallèles, et se raccorde par l'intermédiaire d'une entretoise rétrécie (44) à une collerette de trou de vis (41) d'une branche voisine.

3. Plaquette selon la revendication 1 ou 2, caractérisée en ce que la vis est une vis à tête fraisée bombée (10), à empreinte cruciforme, le fond (50) de l'empreinte présentant une courbure concave.

4. Plaquette avec des vis selon la revendication 3, caractérisée en ce que le corps fileté autotaraudeur (11) de la vis (10) présente un diamètre d'environ 0,8 mm, et la tête un diamètre de 1,6 mm.

5. Plaquette avec des vis selon la revendication 3, caractérisée en ce que la courbure concave présente un rayon d'environ 1,5 mm, et la courbure extérieure convexe de la tête un rayon d'environ 2 mm.

6. Plaquette avec des vis selon l'une quelconque des revendications 3 à 5, caractérisée en ce que le filetage présente, pour un diamètre de 0,8 mm, un pas de 0,25 à 0,35 mm.

7. Plaquette avec des vis selon l'une quelconque des revendications 3 à 6, caractérisée en ce que le diamètre de l'âme de la vis (10) est égal à environ 0,4 mm.

8. Plaquette avec des vis selon l'une quelconque des revendications 3 à 7, dans laquelle les vis à os présentent un filetage autotaraudeur, caractérisé en ce que des méplats (52, 53) sont prévus à proximité de la pointe sur des côtés opposés du corps fileté (11), méplats qui convergent en direction de la pointe (13).

9. Plaquette avec des vis selon la revendication 8, caractérisé en ce qu'il est prévu une pointe conique (13), de préférence avec un angle d'environ 60°.
